(19) Europäisches Patentamt / European Patent Office / Office européen des brevets

(11) **EP 4 616 788 A1**

(12) **EUROPEAN PATENT APPLICATION**
published in accordance with Art. 153(4) EPC

(43) Date of publication:
**17.09.2025 Bulletin 2025/38**

(21) Application number: **23907673.0**

(22) Date of filing: **19.12.2023**

(51) International Patent Classification (IPC):
***A61B 5/00*** (2006.01)

(52) Cooperative Patent Classification (CPC):
**A61B 5/00**

(86) International application number:
**PCT/KR2023/020934**

(87) International publication number:
**WO 2024/136388 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.12.2022 KR 20220181526**

(71) Applicants:
- Hunbiome Co., Ltd.
  **Seoul 08503 (KR)**
- Cosmax, Inc.
  **Hwaseong-si, Gyeonggi-do 18622 (KR)**

(72) Inventors:
- **AN, Yongju**
  **Seoul 08209 (KR)**
- **AHN, Kung**
  **Gunpo-si Gyeonggi-do 15874 (KR)**
- **YUN, Kyeongeui**
  **Cheonan-si Chungcheongnam-do 31184 (KR)**
- **PARK, Myeongsam**
  **Seongnam-si Gyeonggi-do 13486 (KR)**
- **LEE, Donggeol**
  **Seongnam-si Gyeonggi-do 13486 (KR)**
- **KANG, Seunghyun**
  **Seongnam-si Gyeonggi-do 13486 (KR)**

(74) Representative: **Appelt, Christian W.**
**Boehmert & Boehmert
Anwaltspartnerschaft mbB
Pettenkoferstrasse 22
80336 München (DE)**

(54) **DEVICE FOR DETERMINING SKIN TYPE**

(57)    The present invention relates to a device for determining skin type. The device for determining skin type according to an embodiment of the present invention comprises: a color tone sensor for measuring the color tone of a subject's skin; an elasticity sensor for measuring the elasticity of the subject's skin; one or more processors; a memory; and one or more programs stored in the memory and configured to be executed by the one or more processors, wherein the one or more programs include commands that determine a color tone index by changing the color tone data measured by the color tone sensor to a value within a specified range, determine the elasticity index by changing the elasticity data measured by the elasticity sensor to a value within a specified range, and define the skin type of the subject on the basis of the color tone index and the elasticity index.

[Fig.1]

## Description

## Technical Field

The present invention relates to a skin type determination device.

## Background

Conventionally, the Baumann Skin Type Test, which consists of 16 key survey questions, has been used to classify skin types (U.S. Patent Application Publication No. US2014/0018634 A1). The Baumann questionnaire assesses whether a person's skin is oily or dry, sensitive or resistant, pigmented or non-pigmented, and wrinkled or elastic to determine an individual's skin type.

However, the Baumann skin type classification method has reliability issues due to its reliance on self-assessment surveys, and its classification results are overly complex, making it inefficient as a basis for providing customized solutions.

## Technical Problem

The problem to be solved by the present invention is to provide a method for accurately and rapidly determining a relative skin type according to age.

The problems to be solved by the present invention are not limited to those mentioned above, and additional problems not explicitly stated will be clearly understood by those skilled in the art from the descriptions provided below.

## Solution to the Problem

To achieve the above object, a skin type determination device according to an embodiment of the present invention includes a skin tone sensor that measures the skin tone of the subject's skin; an elasticity sensor that measures the elasticity of the subject's skin; one or more processors; memory; and one or more programs stored in the memory and configured to be executed by the one or more processors, wherein the one or more programs include instructions to change the skin tone data measured by the skin tone sensor to a value within a predetermined range to determine a skin tone index, change the elasticity data measured by the elasticity sensor to a value within a predetermined range to determine an elasticity index, and define the skin type of the subject based on the skin tone index and elasticity index.

## Effects of the Invention

According to the skin type determination device of the present invention, it is possible to accurately and rapidly determine a relative skin type while considering age.

The effects of the present invention are not limited to those mentioned above, and additional effects not explicitly stated will be clearly understood by those skilled in the art based on the claims.

## Brief Description of the Drawings

FIG. 1 is a block diagram of a skin type determination device according to an embodiment of the present invention.

FIG. 2 is a graph displaying the results of subjects after normalizing tone data and elasticity data by processors according to an embodiment of the present invention.

FIG. 3 is a graph displaying the results of subjects after normalizing oil content data and moisture content data by processors according to an embodiment of the present invention.

FIG. 4 is a graph showing the distribution of subjects after determining a first evaluation index and a second evaluation index by processors according to an embodiment of the present invention.

FIG. 5a illustrates an example where processors detect a point of inversion in the tone index according to an embodiment of the present invention.

**FIG. 5b** illustrates an example where processors detect a point of inversion in the elasticity index according to an embodiment of the present invention.

**FIG. 5c** illustrates an example where processors detect a point of inversion in the oil index according to an embodiment of the present invention.

**FIG. 5d** illustrates an example where processors detect a point of inversion in the moisture index according to an embodiment of the present invention.

**FIG. 6a** clarifies the table in FIG. 5a by including age and group-specific ratios.

**FIG. 6b** clarifies the table in FIG. 5b by including age and group-specific ratios.

**FIG. 6c** clarifies the table in FIG. 5c by including age and group-specific ratios.

**FIG. 6d** clarifies the table in FIG. 5d by including age and group-specific ratios.

**FIG. 7a** represents the tone values of subjects in a normal distribution curve.

**FIG. 7b** represents the elasticity values of subjects in a normal distribution curve.

**FIG. 7c** represents the moisture values of subjects in a normal distribution curve.

**FIG. 7d** represents the oil content values of subjects in a normal distribution curve.

**FIG. 8a** is a scatter plot distinguishing between a High group and a Low group based on the product of normalized tone data and elasticity data for the **young group** (ages 10-34) according to an embodiment of the present invention.

**FIG. 8b** is a scatter plot distinguishing between a High group and a Low group based on the product of normalized tone data and elasticity data for the **aging1 group** (ages 35-50) according to an embodiment of the present invention.

**FIG. 8c** is a scatter plot distinguishing between a High group and a Low group based on the product of normalized tone data and elasticity data for the **old group** (ages 51-71) according to an embodiment of the present invention.

**FIG.** 9 indicates the distribution positions of subjects without applying the first age weight and the second age weight, marking them in the graph of FIG. 4 according to age groups.

**FIG. 10** is a flowchart illustrating a **skin type determination method** according to an embodiment of the present invention.

**FIG. 11** is a flowchart illustrating a **method for deriving phenotypes by age group.**

## Detailed Description

The advantages and features of the present invention, as well as the methods for achieving them, will become apparent by referring to the embodiments described in detail below in conjunction with the accompanying drawings.
However, the present invention is not limited to the embodiments disclosed below and may be implemented in various different forms, and the embodiments provided herein are merely to ensure the completeness of the disclosure of the present invention and to fully inform those skilled in the art of the scope of the invention, and the present invention is defined only by the scope of the claims, and throughout the specification, the same reference numerals refer to the same components.
Hereinafter, the present invention will be described with reference to the drawings.

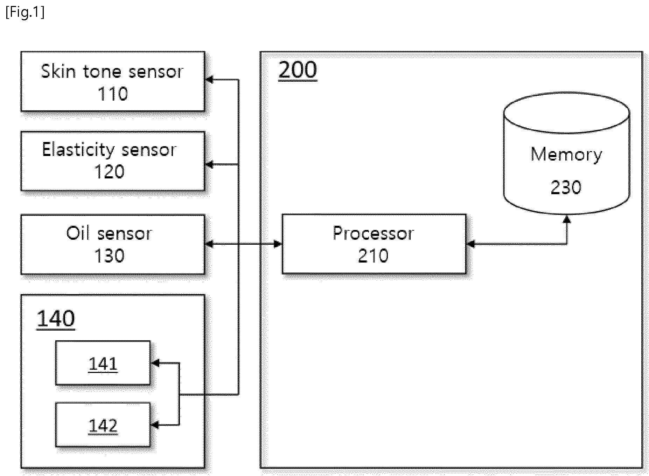

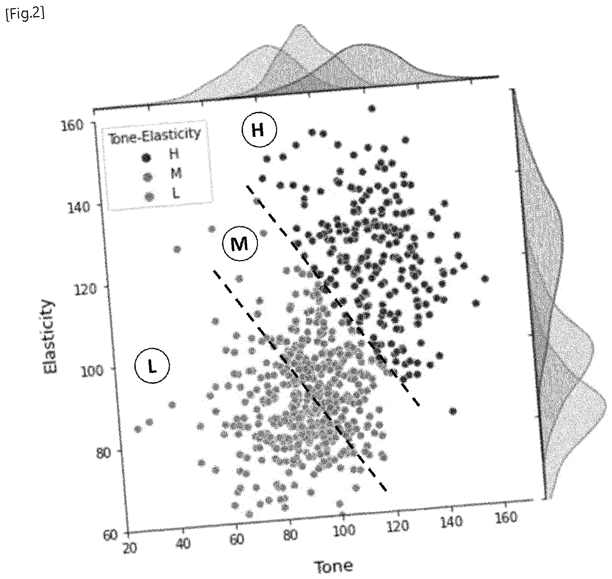

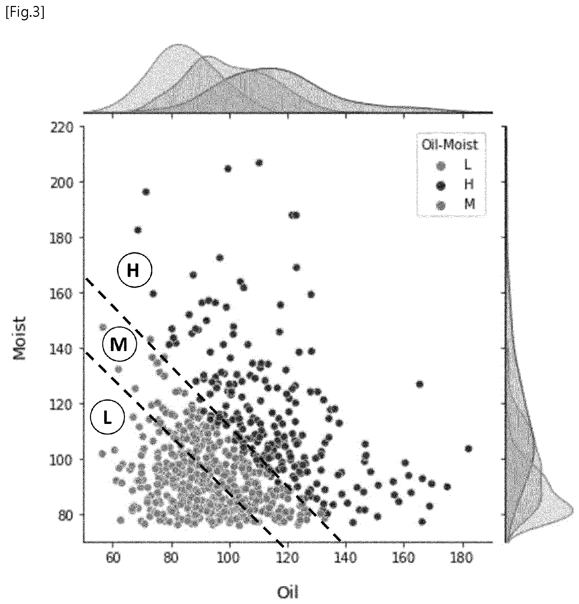

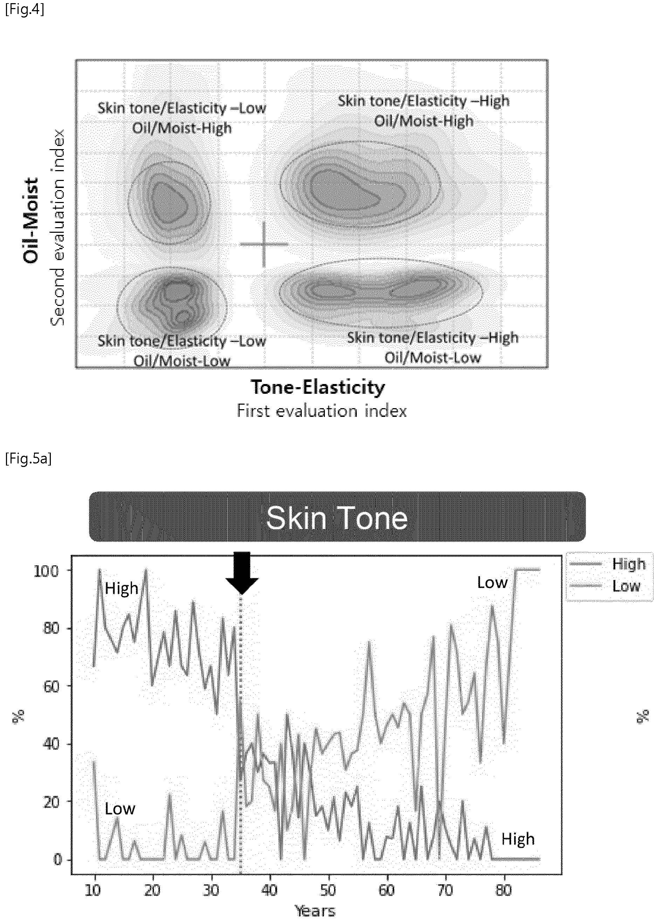

FIG. 1 is a block diagram of a skin type determination device according to various embodiments of the present invention, FIG. 2 is a graph showing the results after normalizing skin tone data and elasticity data using processors (210) according to various embodiments of the present invention, FIG. 3 is a graph displaying oil content data and moisture content data for each subject after normalization by the processors (210) according to various embodiments of the present invention, and FIG. 4 is a graph illustrating the distribution of subjects after determining a first evaluation index and a second evaluation index through the processors (210) according to various embodiments of the present invention.

Referring to FIGS. 1 to 4, the skin type determination device or system according to various embodiments of the present invention includes a skin tone sensor (110) for measuring the skin tone of a subject, an elasticity sensor (120) for measuring the elasticity of the subject's skin, a memory (230) for storing skin tone data measured by the skin tone sensor (110) and elasticity data measured by the elasticity sensor (120), and one or more processors (210) configured to normalize the skin tone data to determine a skin tone value, normalize the elasticity data to determine an elasticity value, and determine a first evaluation index based on the skin tone value and elasticity value to define the subject's skin type.

The skin tone sensor (110) may be a color sensor or an optical sensor, and it detects the color of the skin surface and receives chromatic information from the skin.

The elasticity sensor (120) detects skin elasticity, including contraction, recovery amount, and recovery time, and it may be a distance measurement sensor using laser, ultrasound, or infrared technology, and the elasticity sensor (120) collects information about skin contraction and recovery, while the processor (210) can determine the recovery time.

The memory (230) may optionally include one or more non-transitory computer-readable storage media and may also include high-speed random-access memory (RAM) and optionally comprise non-volatile memory such as one or more magnetic disk storage devices, flash memory devices, or other non-volatile solid-state memory devices.

One or more processors (210) execute or run various software programs and/or sets of instructions stored in memory (230) to perform different functions for the skin type determination device and process data. The processor (210) normalizes the data. Normalization means changing the range of data values to be between 0 and 1. The processor (210) changes all the data values to have the same unit or to a dimensionless number.

A skin type determination device or system according to various embodiments of the present invention includes an oil sensor (130), a moisture sensor (140), an elasticity sensor (120), a skin tone sensor (110), and a main device (200). The main device (200) includes a processor and memory (230). The various sensors (110, 120, 130, 140) and the main device (200) are connected either wired or wirelessly. The main device (200) can be installed on a cloud server. All or part of the components can be a portable mobile device.

According to FIG. 2, the skin tone data and elasticity data are distributed differently for each subject, and according to FIG. 3, the oil data and moisture data are distributed differently for each subject (H: high, M: medium, L: low). The processor (210) can normalize the skin tone data and elasticity data for each subject and store them in memory (230).

The processor (210) determines the first evaluation index. The first evaluation index is an index that integrates the skin tone data and elasticity data. As shown in FIG. 4, the processor (210) determines one first evaluation index based on the skin tone data and elasticity data.

The processor (210) performs operations on two or more variables. An operation refers to a mathematical, logical, or other type of calculation using two or more variables. For example, the processor (210) can perform mathematical operations. Mathematical operations include basic arithmetic (addition, subtraction, multiplication, division), exponentiation, logarithmic operations, differentiation, integration, and so on. The processor (210) can determine the first evaluation index and the second evaluation index, which will be described later, by multiplying the skin tone data and elasticity data.

Alternatively, the processor (210) can perform comparison operations. The processor (210) evaluates at least one of the following based on a predefined standard: 'greater than', 'less than', 'below', or 'above', and returns a predefined value based on the result. In the following, multiplication is used as one example of a preferred operation, but depending on the correlation of data for determining skin type and the advantage in data compression, other mathematical, logical, or comparison operations may also be possible. Therefore, in this invention, 'operation' does not solely refer to multiplication.

A skin type determination device or system according to various embodiments of the present invention includes a skin tone sensor (110) that measures the skin tone of the subject's skin; an elasticity sensor (120) that measures the elasticity of the subject's skin; one or more processors (210); memory (230); and one or more programs stored in memory (230) and configured to be executed by the one or more processors (210). The programs include instructions to change the skin tone data measured by the skin tone sensor (110) to a value within a predetermined range to determine a skin tone value, change the elasticity data measured by the elasticity sensor (120) to a value within a predetermined range to determine an elasticity value, and based on the skin tone value and elasticity value (one preferred example being multiplication), determine a first evaluation index that defines the subject's skin type.

The value within the predefined range can be between 0 and 1. As a specific example, the skin tone data measured by the skin tone sensor (110) can be within the range of 0 to 1. Another specific example is that the elasticity data measured by the elasticity sensor (120) can be within the range of 0 to 1. The processor (210) changes the columns of data to the range of 0 to 1. The processor (210) can perform machine learning.

The processor (210) can standardize the data. Standardization can be performed under the assumption that the data follows a normal distribution (bell-shaped distribution). The processor (210) can transform the data so that its mean is 0 and its standard deviation is 1.

The processor (210) can make the data easier to learn through normalization or standardization. The processor (210) does not focus on any particular column but learns the columns equally. After standardizing and normalizing the data and performing machine learning, the processor (210) can decide whether to standardize or normalize the data, based on the results of both cases.

A skin type determination device or system according to various embodiments of the present invention includes an oil sensor (130) that measures the oil content of the subject's skin; a moisture sensor (140) that measures the moisture content of the subject's skin; one or more processors (210); memory (230); and one or more programs stored in memory (230) and configured to be executed by the one or more processors (210). The programs include instructions to change the oil data measured by the oil sensor (130) to a value within a predefined range to determine the oil value, change the moisture data measured by the moisture sensor (140) to a value within a predefined range to determine the moisture value, and based on the oil and moisture values (one preferred example being multiplication), determine a second evaluation index that defines the subject's skin type.

According to FIG. 2, the oil data and moisture data are distributed differently for each subject (H: high, M: medium, L: low). The processor (210) can normalize the oil and moisture data for each subject and store it in memory (230). The processor (210) can use the oil and moisture data to determine one second evaluation index that integrates oil and moisture.

The processor (210) determines the second evaluation index. The second evaluation index is an index that integrates the oil and moisture data. As shown in FIG. 4, the processor (210) determines a first evaluation index based on the skin tone data and elasticity data (one preferred example being multiplication). The processor (210) can use both the first evaluation index and the second evaluation index to display the subject's skin condition on a single plane.

The moisture sensor (140) includes a first moisture measuring device that measures the transepidermal water loss (TEWL) of the subject's skin, and a second moisture measuring device that measures the hydration (HD) of the subject's skin. The one or more programs include instructions to change the moisture loss and hydration values to values within a predefined range and determine the moisture value using the following formula.

$$M = \frac{HD}{TEWL}$$

Here, M is the moisture value, HD is the hydration of the skin, and TEWL is the transepidermal water loss of the skin.

The processor (210) can integrate the moisture content and moisture loss to determine a single moisture value to represent the skin's moisture status. The processor (210) determines that the lower the transepidermal water loss, the better the skin barrier function is maintained. The processor (210) determines that the lower the moisture loss, the more moisturizing the skin is. The processor (210) determines that the higher the hydration of the skin, the better the skin's moisturizing ability. The processor (210) determines that the higher the moisture value (M), the more moisturizing the skin is.

The first moisture measuring device measures the moisture content of the skin per minute and measures the amount of moisture lost per minute. The transepidermal water loss refers to the amount of moisture lost from the skin barrier located in the stratum corneum, which is the outermost layer of the skin. The second moisture measuring device measures the amount of moisture contained in the skin.

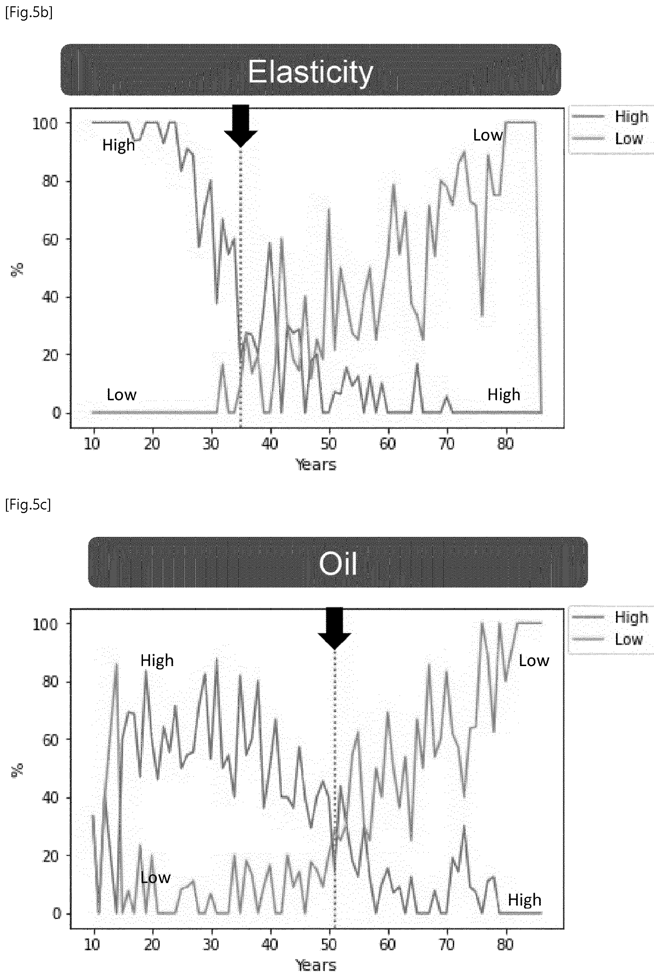

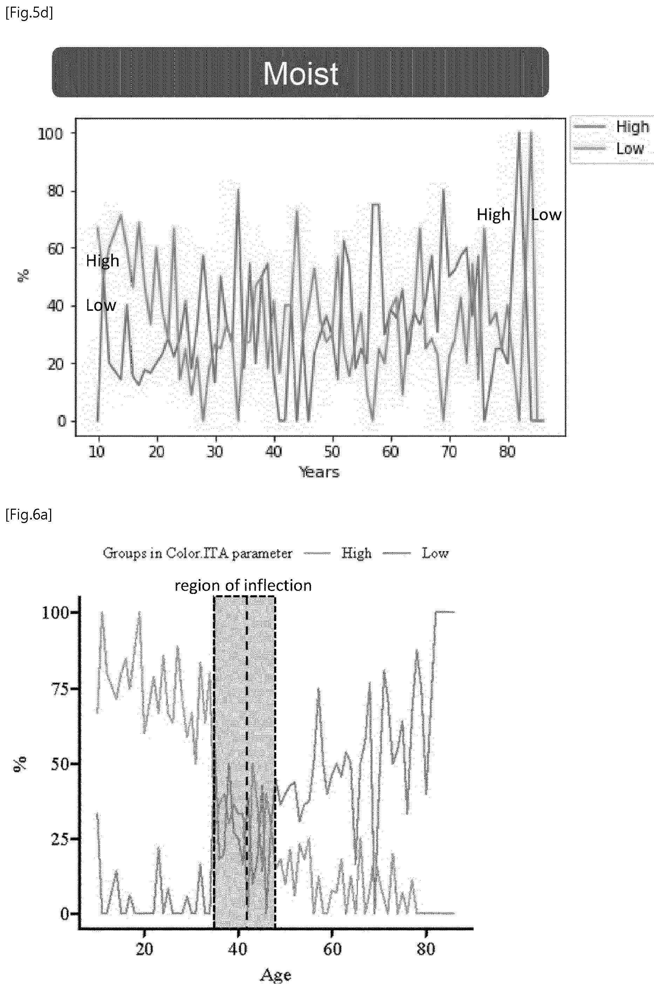

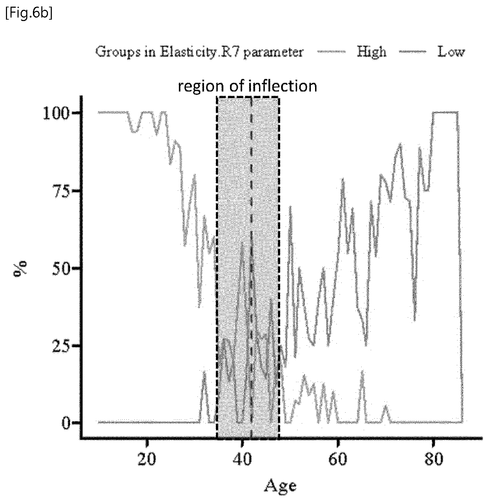

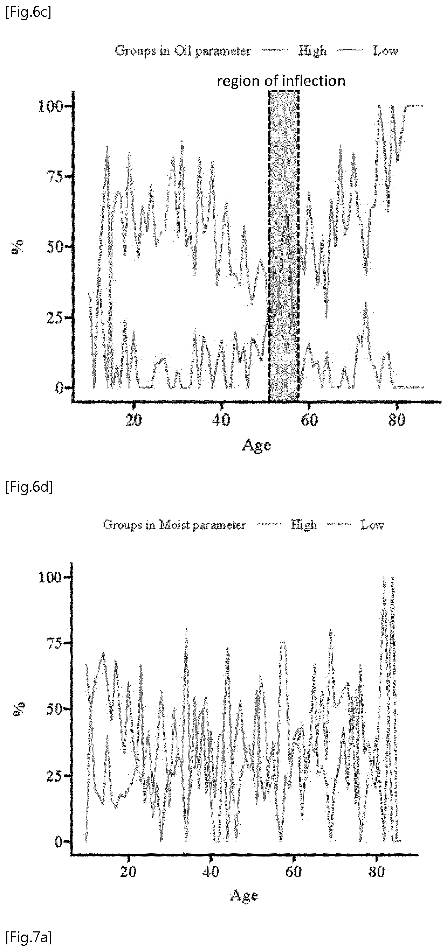

FIG. 5a illustrates an example where the processors (210) of various embodiments of the present invention identify the point where the inversion of the skin tone index occurs. FIG. 5b illustrates an example where the processors (210) of various embodiments of the present invention identify the point where the inversion of the elasticity index occurs. FIG. 5c illustrates an example where the processors (210) of various embodiments of the present invention identify the point where the inversion of the oil index occurs. FIG. 5d illustrates an example where the processors (210) of various embodiments of the present invention identify the point where the inversion of the moisture value occurs. FIG. 6a clarifies the chart in FIG. 5a by adding age and group ratio details. FIG. 6b clarifies the chart in FIG. 5b by adding age and group ratio details. FIG. 6c clarifies the chart in FIG. 5c by adding age and group ratio details. FIG. 6d clarifies the chart in FIG. 5d by adding age and group ratio details.

The skin type determination device according to various embodiments of the present invention includes an oil sensor (130) that measures the oil content of the subject's skin and a moisture sensor (140) that measures the moisture content of the subject's skin.

One or more programs include instructions to quantify individual skin condition data measured by the sensors according to predefined criteria, classify the data into groups, and select similar types of data with group ratio change patterns similar to those of specific age groups to determine the subject's skin condition.

The pattern can be judged based on the similarity of the inversion phenomenon occurring at the age group where the ranking of group ratios changes. The types of data with similar group ratio change patterns may include skin tone data and elasticity data.

Explanation of the meaning of inversion phenomenon.

According to FIG. 5a and FIG. 6a, in the case of the tone value of the subjects, the high and low groups change in proportion at the age of 34, with a complete inversion of the pattern in the late 40s. That is, the high group is the majority under the age of 34, but after 34, the low group gradually becomes the majority.

Again, according to FIG. 5b and FIG. 6b, in the case of the elasticity value of the subjects, the high and low groups change in proportion at the age of 34, with a complete inversion of the pattern in the late 40s. That is, the high group is the majority under the age of 34, but after 34, the low group gradually becomes the majority.

Thus, both the tone value and the elasticity value exhibit inversion phenomena, and the occurrence age ranges are the same. Therefore, the data for the tone value and elasticity value have the same pattern. The tone value and elasticity value are highly correlated.

Therefore, when multiplying the tone value and elasticity value, the characteristics are amplified, making the features of the data more clearly distinguishable.

On the other hand, referring to FIG. 5c and FIG. 6c, the oil value exhibits an inversion phenomenon, but the age range of occurrence is clearly distinct from that of the tone and elasticity values. Referring to FIG. 5d and FIG. 6d, the moisture value does not exhibit an inversion phenomenon. The moisture value expresses an individual's skin condition but has a low correlation with age.

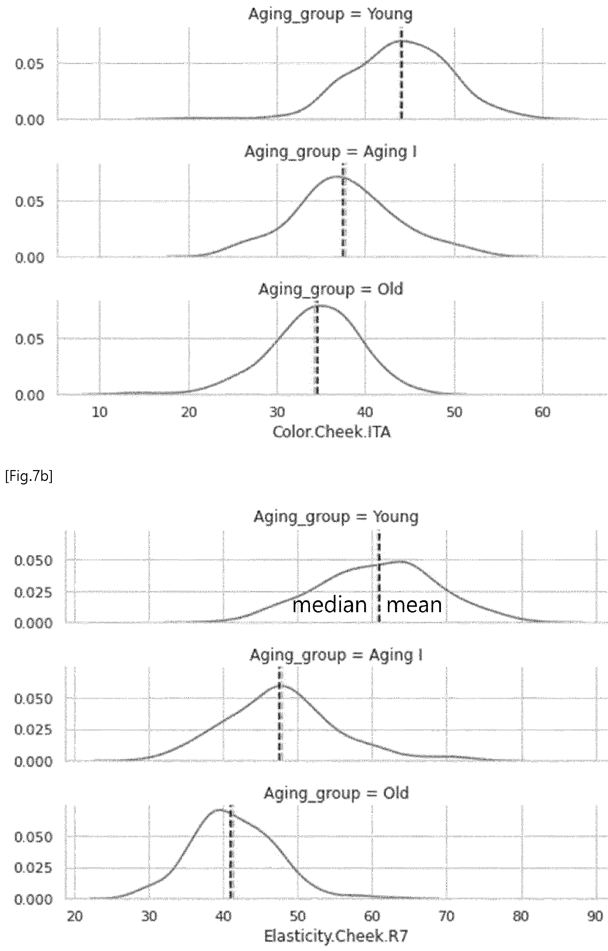

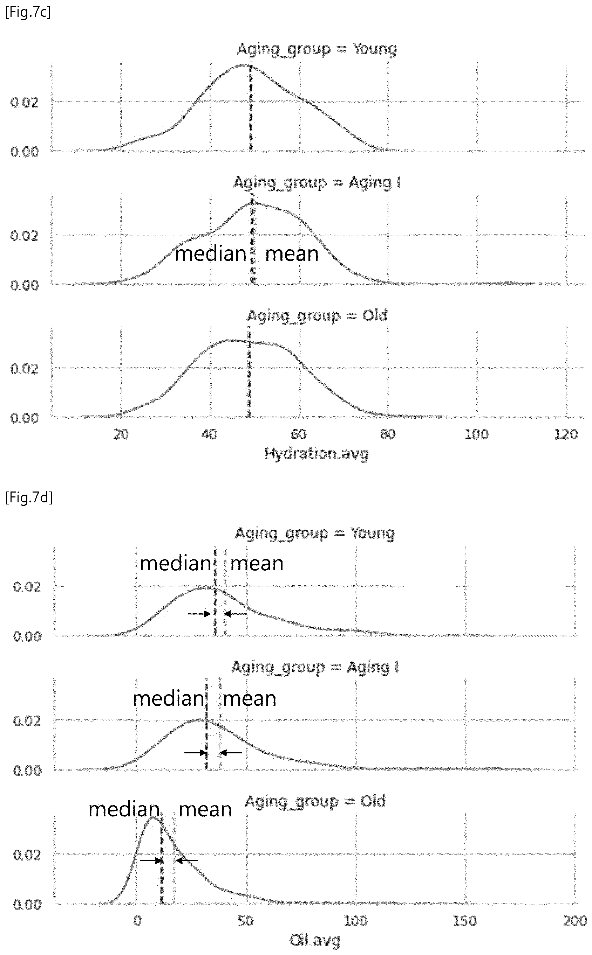

FIG. 7a shows the tone values of the subjects expressed as a normal distribution curve. FIG. 7b shows the elasticity values of the subjects expressed as a normal distribution curve. FIG. 7c shows the moisture values of the subjects expressed as a normal distribution curve. FIG. 7d shows the oil values of the subjects expressed as a normal distribution curve.

One or more programs according to various embodiments of the present invention include instructions to select the remaining data, whose group ratio change pattern is dissimilar, to determine the skin condition of the subject. The remaining data with a dissimilar group ratio change pattern include the oil data measured by the oil sensor (130) and the moisture data measured by the moisture sensor (140).

Referring to FIG. 7a, FIG. 7b, and FIG. 7c, the median and mean values coincide. According to FIG. 7d, the oil value has an asymmetrical distribution or a long-tailed form, indicating that outliers or unusual values are affecting the mean.

There are individuals with abnormally high oil values (at the tail), and it is desirable to reduce the influence of these outliers.

According to various embodiments of the present invention, multiplying the oil value (oil data) by the moisture value (moisture data) can reduce the influence of outliers in the oil data and enhance the pattern of normal data. Thus, the quality of the data and the accuracy of the judgment are increased. Moreover, multiplying the oil value (oil data) by the moisture value (moisture data) can serve as a judgment index that can evaluate an individual's skin condition regardless of age.

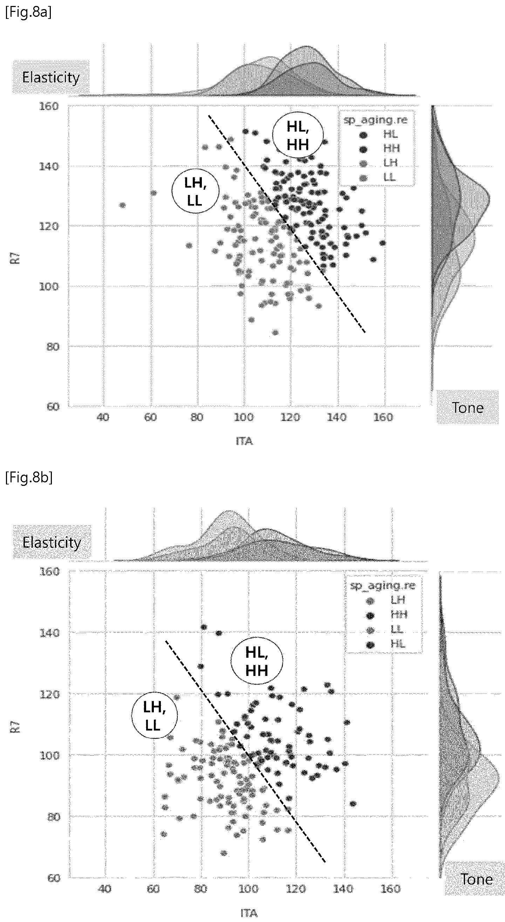

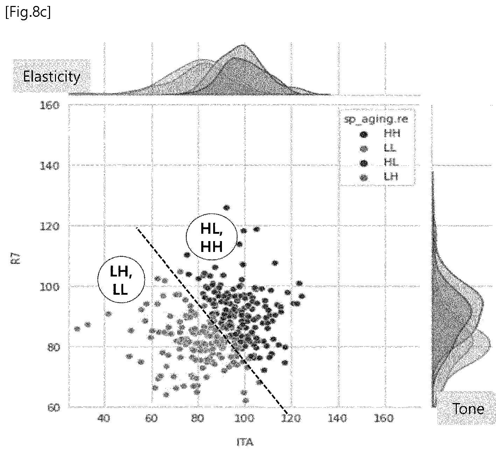

FIG. 8a is a scatter plot showing the distinction between the High group and Low group based on the multiplication of normalized tone and elasticity data from young group (ages 10-34). FIG. 8b shows the same distinction for the aging1 group (ages 35-50). FIG. 8c shows the same distinction for the old group (ages 51-71).

Referring to FIG. 8a, FIG. 8b, and FIG. 8c, if the product of the X-axis value (tone value) and Y-axis value (elasticity value) at any given point exceeds a certain value, it is classified as the High group, and if it is below that value, it is classified as the Low group.

FIG. 8a analyzes the group of relatively young subjects, with the division line (dashed line) for the High and Low groups positioned in the upper-right corner. As you move to FIG. 8b and FIG. 8c, the division line shifts to the lower-left corner. Commonly, the results of the subjects are clustered around the division line. This shows that the multiplication result of the tone value and elasticity value has fewer outliers, making it suitable as a criterion for determining skin condition.

On the other hand, skin condition is absolutely influenced by age, so absolute skin condition judgments without considering age are meaningless. The division line that moves according to age becomes the criterion for relative skin condition judgment considering age. Therefore, the product of the tone value and elasticity value serves as the criterion for determining relative skin condition while considering age.

According to various embodiments of the present invention, the condition of the subjects can be judged relative to their age in four distinct phenotypes.

The processor (210) utilizes the multiplication result of the tone data and elasticity data, as well as the multiplication result of the oil data and moisture data, to classify the subject's skin condition into four groups: HH, HL, LH, and LL. The processor (210) can classify the four groups as follows: HH (both tone*elasticity *and* oil*moisture are high), HL (tone*elasticity *is high but* oil*moisture is low), LL (both tone*elasticity and oil*moisture are low), LH (tone*elasticity *is low but* oil*moisture is high). The four groups exhibit characteristic distributions on a graph.

The skin type determination device according to various embodiments of the present invention can classify the subject's skin type by age group. That is, it can judge relative skin conditions by age group, rather than absolute skin condition. Since age has an absolute influence on skin condition, judgments without considering age may be meaningless.

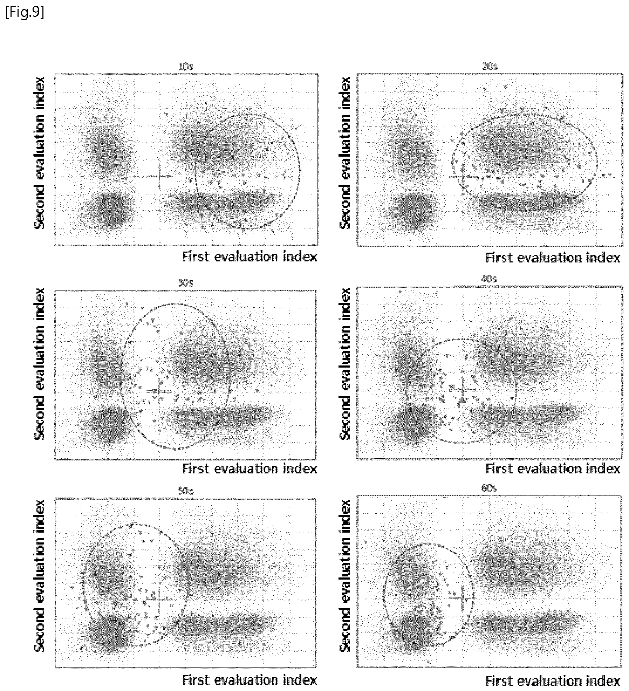

FIG. 9 shows the distribution locations of subjects by age group, plotted on the graph of FIG. 4 without applying the first and second age weights.

The first evaluation index is the product of the tone value and elasticity value, and the second evaluation index is the product of the oil value and moisture value.

Referring to FIG. 9, the result values (inverted triangle shapes) of subject groups in the 10s, 20s, 30s, 40s, 50s, and 60s form groups and gradually move. This shows that the first and second evaluation indices intuitively present the relative results of the subject's skin condition compared to the group of the same age.

According to various embodiments of the present invention, even if classified as LL in the YOUNG group, it could be classified as HH or HL in the OLD group, thus enabling appropriate skin prescriptions based on the group.

The center or boundary of the phenotypic cluster, or the cluster itself, shifts according to age group. After the processor (210) determines the center or cluster characteristics, it can distinguish the relative skin condition of the subject.

One or more programs according to various embodiments of the present invention calculate the first phenotype by applying a predetermined first age weight to the first evaluation index based on the subject's age (a preferred example is a multiplication operation).

One or more processors (210) are configured to determine the second phenotype by applying a predetermined second age weight to the second evaluation index based on the subject's age (a preferred example is a multiplication operation).

For example, the processor (210) can classify subjects into three groups: the Young group (under 34 years old), the Aging I group (from 35 to 50 years old), and the Old group (over 51 years old). The first and second age weights may vary based on gender. The processor (210) can apply different first and second age weights based on age groups.

The processor (210) can determine the first and second age weights differently depending on age through machine learning. The first and second age weights may be predefined. These weights can be stored in memory (230).

The processor (210) can determine the age at which an inversion phenomenon occurs by comparing the tone index of multiple collected subjects. For example, the processor (210) may determine that, for subjects under 35 years old, most have a tone index exceeding 0.5, while for subjects over 35 years old, most have a tone index below 0.5 (in FIGS. 5a, 5b, 5c, and 5d, "high" is used to indicate most, and "low" is used for few, with arrows marking the inversion points).

In this case, the processor (210) determines that the inversion phenomenon occurs at 35 years old for the tone index. Therefore, the processor (210) may set the first age weight to 1 for those over 35 years old and to 0.7 for those under 35 years old. Similarly, the processor (210) may determine the inversion point for the elasticity index to be 35 years old.

The processor (210) can determine the age at which an inversion phenomenon occurs by comparing the oil values of multiple collected subjects. For example, the processor (210) may classify subjects into three groups: under 30 years old, 30 to 50 years old, and over 50 years old, and determine the second age weight accordingly.

For example, the processor (210) may determine that subjects under 15 years old mostly have oil values exceeding 0.5, subjects between 15 and 50 years old mostly have oil values below 0.5, and subjects over 50 years old mostly have oil values exceeding 0.5.

In this case, the processor (210) determines that inversion phenomena occur at ages 15 and 50. The processor (210) applies different second age weights around these ages.

The processor (210) considers the relative differences due to age by applying the first and second age weights. It determines the subject's skin condition relative to the same age group by applying the first and second age weights.

Referring to FIG. 6, the skin condition of subjects is clustered within a certain range for each age group. However, the skin condition should be judged relatively within the same age group. Therefore, the processor (210) needs to consider the distribution by age.

For example, if a subject in their teens is judged to have better objective skin condition than a subject in their 70s, but less elasticity compared to other subjects in the same age group, the processor (210) may assign a symbol indicating that the first phenotype for the teen subject is low, insufficient, or poor compared to their age group. Conversely, the processor (210) may assign a symbol indicating that the first phenotype for the 70s subject is high, sufficient, or excellent compared to their age group.

The processor (210) can determine the second age weight and the second phenotype in the same way as the first age weight and the first phenotype.

According to an embodiment, the memory (230) may store information about the first and second age weights in advance.

The skin type determination device according to various embodiments of the present invention includes a color tone sensor (110), an elasticity sensor (120), a moisture sensor (140), an oil sensor (130), and one or more processors (210). The device comprises the steps of: measuring the color tone, elasticity, oil, and moisture of the subject's skin; and having one or more processors (210) determine the first and second evaluation indices. The first evaluation index is determined by the processor (210) changing the color tone data to a value within a predetermined range, determining the color tone value, changing the elasticity data to a value within a predetermined range, determining the elasticity value, and calculating (a preferred example is multiplication) the color tone and elasticity values; the second evaluation index is determined by the processor (210) changing the oil data to a value within a predetermined range, determining the oil value, changing the moisture data to a value within a predetermined range, determining the moisture value, and calculating (a preferred example is multiplication) the oil and moisture values.

The skin type determination device or system according to various embodiments of the present invention includes: an oil sensor (130) for measuring the oil of the subject's skin; a moisture sensor (140) for measuring the moisture of the subject's skin; a memory (230) for storing the oil data measured by the oil sensor (130) and the moisture data measured by the moisture sensor (140); and one or more processors (210). The processor (210) is configured to normalize the oil data to determine the oil value, normalize the moisture data to determine the moisture value, and determine the second evaluation index that defines the subject's skin type based on (a preferred example is multiplication) the oil and moisture values.

The skin type determination device or system according to various embodiments of the present invention includes: a color tone sensor (110) for measuring the color tone of the subject's skin; an elasticity sensor (120) for measuring the elasticity of the subject's skin; one or more processors (210); memory (230); and one or more programs stored in memory (230) and executed by one or more processors (210). The programs include instructions that change the color tone data measured by the color tone sensor (110) to a value within a predetermined range to determine the color tone value, change the elasticity data measured by the elasticity sensor (120) to a value within a predetermined range to determine the elasticity value, and calculate (a preferred example is multiplication) the color tone and elasticity values to determine the first evaluation index that defines the subject's skin type.

The values within the predetermined range may be between 0 and 1. One or more programs calculate the first phenotype by applying the predetermined first age weight to the first evaluation index (a preferred example is multiplication) based on the subject's age, and store the first and second phenotypes in memory (230).

The skin type determination device or system according to various embodiments of the present invention includes: a moisture sensor (140) for measuring the moisture of the subject's skin; one or more processors (210); memory (230); and one or more programs stored in memory (230) and configured to be executed by one or more processors (210). The programs include instructions that change the moisture data measured by the moisture sensor (140) to a value within a predetermined range to determine the moisture value. The moisture sensor (140) includes: a first moisture measuring device for measuring the moisture loss of the subject's skin; and a second moisture measuring device for measuring the moisture content (hydration) of the subject's skin. The programs change the moisture loss and moisture content to values within a predetermined range and include instructions that determine the moisture value using the following formula to assess the skin condition.

$$M = \frac{HD}{TEWL}$$

Here, M is the moisture value, HD is the moisture content, and TEWL is the moisture loss.

The skin type determination device or system according to various embodiments of the present invention includes: a color sensor (110) for measuring the skin tone of the subject; an elasticity sensor (120) for measuring the elasticity of the subject's skin; a sebum sensor (130) for measuring the sebum of the subject's skin; a moisture sensor (140) for measuring the moisture of the subject's skin; memory (230) for storing the color data measured by the color sensor (110) and the elasticity data measured by the elasticity sensor (120); and one or more processors (210).

One or more processors (210) are configured to: change the color data to a value within a predetermined range to determine the color value; change the elasticity data to a value within a predetermined range to determine the elasticity value; calculate the first evaluation index by multiplying the color value and the elasticity value to determine the subject's skin type; change the sebum data to a value within a predetermined range to determine the sebum value; change the moisture data to a value within a predetermined range to determine the moisture value; and calculate the second evaluation index based on the sebum value and the moisture value (preferably using multiplication) to determine the subject's skin type.

The memory (230) stores a standardization or normalization program executed by one or more processors (210) so that the data can be changed to a value within the predetermined range.

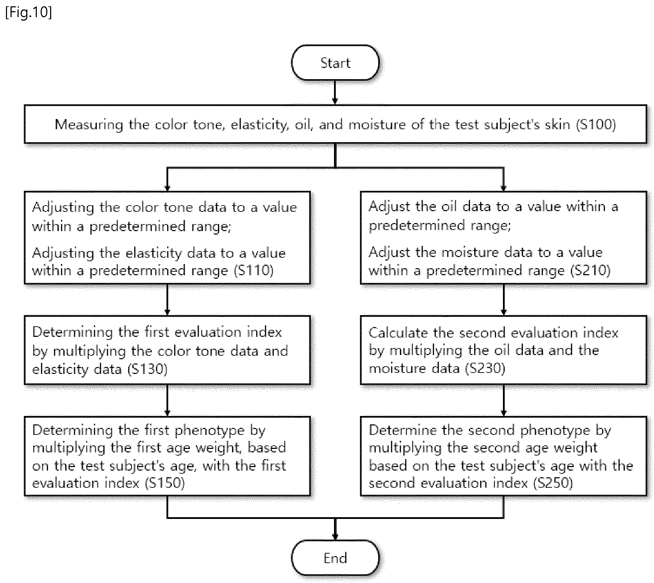

FIG. 10 is a flowchart of the skin type determination method according to various embodiments of the present invention.

Referring to FIG. 10, the skin type determination method according to various embodiments of the present invention comprises: measuring the skin tone, elasticity, sebum, and moisture of the subject's skin using the skin type determination device with the color sensor (110), elasticity sensor (120), moisture sensor (140), and sebum sensor (130) (S100); and one or more processors (210) determining the first and second evaluation indexes. The first evaluation index comprises the processor (210) changing the color data to a value within a predetermined range to determine the color value, changing the elasticity data to a value within a predetermined range to determine the elasticity value (S110), and calculating the first evaluation index by multiplying the color value and elasticity value (S130). The second evaluation indexcomprises the processor (210) changing the sebum data to a value within a predetermined range to determine the sebum value, changing the moisture data to a value within a predetermined range to determine the moisture value (S210), and calculating the second evaluation index by multiplying the sebum value and moisture value (S230).

According to various embodiments of the present invention, the processor (210) performs the step of determining the first phenotype by multiplying the first evaluation index by a first age-weighting based on the subject's age (S150). According to various embodiments of the present invention, the processor (210) performs the step of determining the second phenotype by multiplying the second evaluation index by a second age-weighting based on the subject's age (S250). One or more processors (210) can change the moisture loss and moisture content to values within a predetermined range and use them to determine the moisture value. The formula for determining the moisture value is as described earlier.

In various embodiments of the present invention, the skin type determination device and/or system can display the first and second phenotypes by adding an age symbol rather than multiplying with age weights as described above.

$$\text{Skin type} \begin{cases} H\begin{pmatrix} H \\ L \end{pmatrix} \\ L\begin{pmatrix} H \\ L \end{pmatrix} \end{cases}$$

$$\underline{1^{st} \text{ phenotype}} \quad \underline{2^{nd} \text{ phenotype}}$$

The memory (230) can store the subject's skin condition categorized into phenotypes such as H/H, H/L, L/H, L/L. In the phenotype, the prefix "H" or "L" can indicate that the skin's color tone and elasticity are high or low, or excellent or lacking. In the phenotype, the suffix "H" or "L" can indicate that the sebum and moisture are high or low, or excellent or lacking. The phenotype is used as data for improving the subject's skin condition. The previously explained content can also be summarized in the following table and formula.

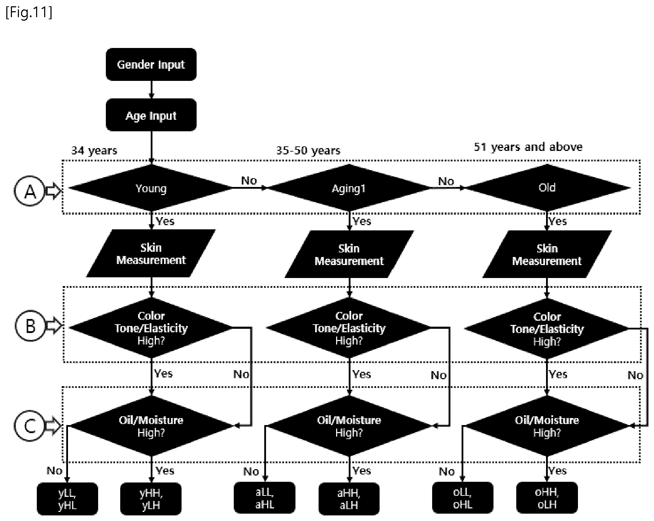

Referring to Figure 11, Step A is related to the age range derivation algorithm (Female & 5 parameters, N=775).

There can be various criteria for high and low age, but in the above embodiment, "young" refers to individuals 34 years or younger, "Aging1" refers to individuals aged 35 to 50, and "Old" refers to individuals 51 years or older.

Then, using the sensors, five parameters-color, elasticity, sebum, moisture, and moisture loss-are measured. In Step B of Figure 11, high and low values are distinguished based on color and elasticity values (Clinical data & Survey, N=705). In Step C of Figure 11, high and low values are distinguished based on sebum and moisture values.

The formulas for standardizing the various data according to the present invention are as follows.

**SS Function: Standard Scalar (mean=100,std=20)**

$$SS(x) = \frac{20 \times (x - x_{mean})}{x_{std}} + 100$$

The above formula is intended to transform the data into a value within the range of 0 to 200.

**Tone and Elasticity value (TE$_{ss}$)**

$$TE_{ss} = SS(Color.ITA_{ss} \times Elasticity.R7_{ss})$$
$$= SS\{SS(Color.Cheek.ITA) \times SS(Elasticity.Cheek.R7)\}$$

The above formula expresses the multiplication of standardized or normalized data related to color tone and elasticity.

**Oil and Moist value (OM$_{ss}$)**

$$OM_{ss} = SS(Oil_{ss} \times Moist_{ss}) = SS\{SS(Oil_{mean}) \times SS(Moist)\}$$

$$= SS\left\{SS(Oil_{mean}) \times SS(\frac{Hydration_{mean}}{TEWL_{mean}})\right\}$$

The above formula expresses the multiplication of standardized or normalized data related to oil and moisture.

$$Oil_{mean} = \frac{Oil.Forehead + Oil.Nose + Oil.Cheek}{3}$$

The above formula expresses the formula for deriving oil data, where the oil is collected from the forehead, nose, and cheeks, and the average value is used.

$$Hydration_{mean} = \frac{Hydration.Forehead + Hydration.Cheek}{2}$$

The above formula expresses the formula for deriving moisture content data, where moisture is collected from the forehead and cheeks, and the average value is used.

$$TEWL_{mean} = \frac{TEWL.Forehead + TEWL.Cheek}{2}$$

The above formula expresses the formula for deriving moisture loss data, where moisture is collected from the forehead and cheeks, and the average value is used.
The method for categorizing aging groups is explained as follows.

$$GRP(x_{ss}) = \begin{cases} L, x_{ss} \leq Q1 \\ M, Q1 < x_{ss} \leq Q3 \\ H, x_{ss} < Q3 \end{cases}$$

$$Q1 = Quantile(x_{ss}, 1), Q3 = Quantile(x_{ss}, 3)$$

The quantile is divided into three groups: the middle group (grey zone), and the remaining upper (H) and lower (L) groups are distinctly separated using this method.
To explain the formula above, first, for color, elasticity, sebum, and moisture SS(x), the GRP(xss) function is executed.

GRP(Color.ITA$_{ss}$), GRP(Elasticity.R7$_{ss}$),
GRP(Oil$_{ss}$), GRP(Moist$_{ss}$).

Next, the age is extracted.
The percent (%) for GRP(x) by age is checked based on clinical measurement values. Then, the age at which the sample ratio of the H type becomes lower than that of the L type is identified.
The derived example is as follows.

$Age_{ITA}$ = 35, $Age_{R7}$ = 36, $Age_{Oil}$ = 51

$$AgingGroup = \begin{cases} Young, 10 \leq Age \leq 34 \\ Aging\,I, 35 < Age \leq 50 \\ Old, 51 \leq Age \end{cases}$$

The following formula indicates that values above the specified threshold are labeled as high, and values below the threshold are labeled as low.

$$SP(x) = \begin{cases} L, & x < x_{mean} \\ H, & x > x_{mean} \end{cases}$$

The following describes that the phenotype related to tone-elasticity (TE) can be expressed as either H or L, as mentioned above.

$$TE_{group} = SP(TE_{ss})$$

The following describes that the phenotype related to oil-moisture (OM) can be expressed as either H or L, as mentioned above.

$$OM_{group} = \begin{cases} SP(OM_{ss}), & TE_{group} = H \\ SP(OM_{ss}), & TE_{group} = L \end{cases}$$

The following describes the group corresponding to age along with the phenotype related to color-tone and elasticity (TE). In the example below, the subject belongs to the Young group.

$$Young.TE_{group} = TE_{group}(TE_{ss}\,in\,Young\,samples)$$

The following describes the group corresponding to age along with the phenotype related to oil-moisture (OM). In the example below, the subject belongs to the Young group.

$$Young.OM_{group} = \begin{cases} SP(OM_{ss}\,in\,Young\,samples), & Young.TE_{group} = H \\ SP(OM_{ss}\,in\,Young\,samples), & Young.TE_{group} = L \end{cases}$$

Information about the color tone-elasticity (TE), oil-moisture (OM), and age can be expressed as follows.

$$Young.KSC = Young.TE_{group} + Young.OM_{group}$$

$$KSC \begin{cases} H\begin{pmatrix} H \\ L \end{pmatrix} \\ L\begin{pmatrix} H \\ L \end{pmatrix} \end{cases}$$

$$\underline{\text{TE type}} \quad \underline{\text{OM type}}$$

The effects of the present invention are described as follows.

According to the present invention, the processor (210) can obtain the first evaluation index and the second evaluation index by performing multiplication operations on the four types of data.

In addition, according to the present invention, the processor (210) can group the test subjects by determining the first and second evaluation indexes, and the data can be utilized for machine learning after undergoing normalization or standardization.

Furthermore, the processor (210) according to the present invention allows for relative evaluation that takes age into account by applying the first and second age weights to the color tone, elasticity, oil, and moisture data.

Also, the processor (210) according to the present invention can objectively evaluate the skin condition by processing only the four types of data. Elasticity is highly correlated with pores and wrinkles. Therefore, the first and second phenotypes described below can be seen as encompassing six indicators, including pores and wrinkles.

Moreover, according to the present invention, the processor (210) can independently evaluate the moisture condition of the skin using the moisture value without considering age.

Although the present invention has been described and illustrated in the preferred embodiments above, the invention is not limited to the specific embodiments described, and various modifications can be made by those skilled in the art without departing from the spirit of the present invention, as defined in the claims. Such modifications should not be understood individually from the technical ideas or prospects of the present invention.

## Claims

1. A skin type determination device comprising:

   A color tone sensor for measuring the color tone of the test subject's skin;
   An elasticity sensor for measuring the elasticity of the test subject's skin;
   One or more processors;
   A memory; and
   One or more programs stored in the memory and configured to be executed by the one or more processors, wherein the one or more programs include:

   Instructions for changing the color tone data measured by the color tone sensor to a value within a predetermined range to determine a color tone index;
   Instructions for changing the elasticity data measured by the elasticity sensor to a value within a predetermined range to determine an elasticity index; and
   Instructions to determine the skin type of the test subject based on the color tone index and the elasticity index.

2. The skin type determination device of claim 1, wherein the value within the predetermined range is between 0 and 1.

3. The skin type determination device of claim 1, wherein the one or more programs are configured to:

   Calculate a first evaluation index based on the test subject's age and a first age weight; and
   Determine the first phenotype based on the color tone index and the elasticity index.

4. The skin type determination device of claim 1, further comprising:

   An oil sensor for measuring the oil content of the test subject's skin; and
   A moisture sensor for measuring the moisture content of the test subject's skin;

The one or more programs further including instructions to:

Quantify individual skin condition data measured by the sensors based on a predetermined standard to classify groups;
Select types of data whose group ratio change patterns with age are similar, to determine the test subject's skin condition.

5. The skin type determination device of claim 4, wherein the pattern is based on the similarity of the age ranges in which an inversion phenomenon occurs, where the ranking of group ratios is reversed.

6. The skin type determination device of claim 4, wherein the types of data with similar group ratio change patterns include the tone data and the elasticity data.

7. The skin type determination device of claim 6, wherein the one or more programs further include instructions to select data with dissimilar group weight change patterns and determine the test subject's skin condition based on this data.

8. The skin type determination device of claim 7, wherein the data with dissimilar group weight change patterns are the oil data measured by the oil sensor and the moisture data measured by the moisture sensor.

9. The skin type determination device of claim 4, wherein the one or more programs include:

Converting the sebum data to a value within a predetermined range to determine a sebum index,
Converting the moisture data to a value within a predetermined range to determine a moisture index, and
Determining the test subject's skin type based on the sebum index and the moisture index.

10. A skin type determination device comprising:

An oil sensor for measuring the oil content of the test subject's skin;
A moisture sensor for measuring the moisture content of the test subject's skin;
A memory for storing oil data measured by the oil sensor and moisture data measured by the moisture sensor; and
One or more processors, wherein the one or more processors:

Normalize the oil data to determine an oil index;
Normalize the moisture data to determine a moisture index;
Calculate the product of the oil index and the moisture index to determine the test subject's skin type.

11. The skin type determination device of claim 10, wherein the memory stores a program executed by the one or more processors to standardize or normalize the measured test subject's individual data to a value within a predetermined range.

12. A skin type determination device comprising:

A sebum sensor for measuring the sebum of the test subject's skin;
A moisture sensor for measuring the moisture of the test subject's skin;
A color tone sensor for measuring the color tone of the test subject's skin;
An elasticity sensor for measuring the elasticity of the test subject's skin;
One or more processors;
A memory; and
One or more programs stored in the memory and configured to be executed by the one or more processors, wherein the one or more programs include the following instructions:

Change the sebum data measured by the sebum sensor to a value within a predetermined range to determine a sebum index;
Change the moisture data measured by the moisture sensor to a value within a predetermined range to determine a moisture index;
Change the color tone data measured by the color tone sensor to a value within a predetermined range to determine a color tone index;
Change the elasticity data measured by the elasticity sensor to a value within a predetermined range to

EP 4 616 788 A1

determine an elasticity index;
Perform a multiplication operation on the color tone index and the elasticity index to determine the skin type of the test subject; and
Perform a multiplication operation on the sebum index and the moisture index to determine the skin type of the test subject.

13. The skin type determination device of claim 12, wherein the one or more programs include:

Determining a first phenotype by multiplying a first age weight based on the test subject's age with the first evaluation index, which is obtained by multiplying the color tone index and the elasticity index;
Determining a second phenotype by multiplying a second age weight based on the test subject's age with the second evaluation index, which is obtained by multiplying the oil index and the moisture index;
Storing the first phenotype and the second phenotype in the memory.

14. The skin type determination device of claim 12, wherein:

The moisture sensor includes:

A first moisture measurement device for measuring the test subject's transepidermal water loss (TEWL);
A second moisture measurement device for measuring the test subject's hydration (HD);

The one or more programs:

Adjust the moisture loss and moisture content to values within a predetermined range;
Determine the moisture index (M) using the following formula:

$$M = \frac{HD}{TEWL}$$

where M is the moisture index, HD is the hydration of the skin, and TEWL is the transepidermal water loss of the skin.

15. The skin type determination device of claim 12, wherein the memory stores a program executed by the one or more processors to standardize or normalize the measured test subject's individual data to a value within a predetermined range.

[Fig.1]

[Fig.2]

[Fig.3]

[Fig.4]

**Oil-Moist**
Second evaluation index

Skin tone/Elasticity –Low
Oil/Moist-High

Skin tone/Elasticity –High
Oil/Moist-High

Skin tone/Elasticity –Low
Oil/Moist-Low

Skin tone/Elasticity –High
Oil/Moist-Low

**Tone-Elasticity**
First evaluation index

[Fig.5a]

Skin Tone

[Fig.5b]

Elasticity

[Fig.5c]

Oil

[Fig.5d]

[Fig.6a]

[Fig.6b]

Groups in Elasticity.R7 parameter ----- High ----- Low

region of inflection

[Fig.6c]

Groups in Oil parameter — High — Low

[Fig.6d]

Groups in Moist parameter — High — Low

[Fig.7a]

Aging_group = Young

Aging_group = Aging I

Aging_group = Old

Color.Cheek.ITA

[Fig.7b]

Aging_group = Young

median ┊ mean

Aging_group = Aging I

Aging_group = Old

Elasticity.Cheek.R7

[Fig.7c]

[Fig.7d]

[Fig.8a]

[Fig.8b]

[Fig.8c]

[Fig.9]

[Fig.10]

```
                              ┌─────────────┐
                              │    Start    │
                              └──────┬──────┘
                                     │
                                     ▼
┌────────────────────────────────────────────────────────────────────────────┐
│    Measuring the color tone, elasticity, oil, and moisture of the           │
│    test subject's skin (S100)                                                │
└──────────────────────────────────┬─────────────────────────────────────────┘
                 ┌──────────────────┴────────────────────┐
                 ▼                                        ▼
┌──────────────────────────────────┐    ┌──────────────────────────────────┐
│ Adjusting the color tone data to  │    │ Adjust the oil data to a value    │
│ a value within a predetermined    │    │ within a predetermined range;     │
│ range;                            │    │                                   │
│ Adjusting the elasticity data to  │    │ Adjust the moisture data to a     │
│ a value within a predetermined    │    │ value within a predetermined      │
│ range (S110)                      │    │ range (S210)                      │
└──────────────────┬────────────────┘    └──────────────────┬────────────────┘
                   ▼                                          ▼
┌──────────────────────────────────┐    ┌──────────────────────────────────┐
│ Determining the first evaluation  │    │ Calculate the second evaluation   │
│ index by multiplying the color    │    │ index by multiplying the oil data │
│ tone data and elasticity data     │    │ and the moisture data (S230)      │
│ (S130)                            │    │                                   │
└──────────────────┬────────────────┘    └──────────────────┬────────────────┘
                   ▼                                          ▼
┌──────────────────────────────────┐    ┌──────────────────────────────────┐
│ Determining the first phenotype   │    │ Determine the second phenotype    │
│ by multiplying the first age      │    │ by multiplying the second age     │
│ weight, based on the test         │    │ weight based on the test          │
│ subject's age, with the first     │    │ subject's age with the second     │
│ evaluation index (S150)           │    │ evaluation index (S250)           │
└──────────────────┬────────────────┘    └──────────────────┬────────────────┘
                   └──────────────────┬─────────────────────┘
                                      ▼
                              ┌─────────────┐
                              │    End      │
                              └─────────────┘
```

[Fig.11]

## INTERNATIONAL SEARCH REPORT

| International application No. |
|---|
| **PCT/KR2023/020934** |

| A. | CLASSIFICATION OF SUBJECT MATTER |
|---|---|

**A61B 5/00**(2006.01)i

According to International Patent Classification (IPC) or to both national classification and IPC

| B. | FIELDS SEARCHED |
|---|---|

Minimum documentation searched (classification system followed by classification symbols)

A61B 5/00(2006.01); A45D 44/00(2006.01); A61B 5/053(2006.01); A61B 5/107(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 색조 센서(tone sensor), 탄력 센서(elasticity sensor), 색조 지표(tone index), 탄력 지표(elasticity index), 수분 센서(moisture sensor), 유분 센서(oil sensor), 피부타입(skin type)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2022-0142725 A (DERMAPRO) 24 October 2022 (2022-10-24)<br>See paragraphs [0029]-[0148] and figures 1-3. | 1-15 |
| Y | KR 10-2017-0066087 A (LG ELECTRONICS INC.) 14 June 2017 (2017-06-14)<br>See paragraphs [0051]-[0164] and figures 1-3. | 1-15 |
| A | KR 10-2020-0094236 A (BEULA. CO., LTD. et al.) 07 August 2020 (2020-08-07)<br>See paragraphs [0034]-[0111] and figures 2-7. | 1-15 |
| A | KR 10-2014-0067597 A (AMOREPACIFIC CORPORATION) 05 June 2014 (2014-06-05)<br>See paragraphs [0025]-[0042]. | 1-15 |
| A | KR 10-2019-0091857 A (KONG, Seong Jin) 07 August 2019 (2019-08-07)<br>See paragraphs [0029]-[0066] and figures 2-4. | 1-15 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **25 March 2024** | **26 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/020934**

| Patent document cited in search report | | | Publication date (day/month/year) | Patent family member(s) | | | Publication date (day/month/year) |
|---|---|---|---|---|---|---|---|
| KR | 10-2022-0142725 | A | 24 October 2022 | KR | 10-2603356 | B1 | 17 November 2023 |
| KR | 10-2017-0066087 | A | 14 June 2017 | KR | 10-2523871 | B1 | 20 April 2023 |
| KR | 10-2020-0094236 | A | 07 August 2020 | None | | | |
| KR | 10-2014-0067597 | A | 05 June 2014 | CN | 104640503 | A | 20 May 2015 |
| | | | | CN | 104640503 | B | 10 May 2017 |
| | | | | KR | 10-2121552 | B1 | 11 June 2020 |
| | | | | WO | 2014-084602 | A1 | 05 June 2014 |
| KR | 10-2019-0091857 | A | 07 August 2019 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140018634 A1